# EUROPEAN PATENT APPLICATION

(11) **EP 1 437 408 A1**
(43) Date of publication of application: **14.07.2004**
(21) Application number: 02763036.7
(22) Date of filing: 17.09.2002
(51) Int. Cl.: C12N 15/11, C07H 21/00, A61K 31/712, A61P 31/14

(54) **NOVEL ANTISENSE OLIGONUCLEOTIDE DERIVATIVES AGAINST HEPATITIS C VIRUS**

(30) Priority: 17.09.2001 JP 2001282111
(71) Applicant: Imanishi, Takeshi, Nara-shi, Nara 631-0045 (JP)
(72) Inventor: IMANISHI, Takeshi, Nara-shi, Nara 631-0045 (JP); OBIKA, Satoshi, Takatsuki-shi, Osaka 569-1022 (JP); OHSUGI, Yoshiyuki, Chuo-ku, Tokyo 104-8301 (JP)
(74) Representative: VOSSIUS & PARTNER
(86) International application number: PCT/JP2002/009505
(87) International publication number: WO 2003/025173

(57) **Abstract**

An antisense oligonucleotide derivative against HCV is provided which contains one or more nucleotide analogue units having a modified sugar portion and represented by the following general formula 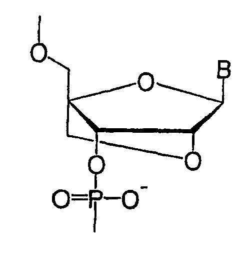 where B denotes a pyrimidine or purine nucleic acid base or an analogue thereof.

The derivative of the present invention is an antisense against hepatitis C virus (HCV) gene, binds to HCV-RNA with high affinity within cells, can control and inhibit the expression of its gene with high efficiency, and shows high resistance to nucleases.

The BNA antisense oligonucleotide of the present invention is also effective in an antisense method targeting HCV, no matter what secondary structures, such as loops or stems, in a target RNA nucleic acid are.

## Description

### [TECHNICAL FIELD]

This invention relates to novel antisenses against hepatitis C virus (HCV). More particularly, the invention relates to novel antisenses against HCV, which comprise oligonucleotide derivatives containing one or more nucleotide analogue units having a modified sugar portion.

### [BACKGROUND ART]

In 1978, it was reported for the first time that antisense molecules inhibited infection by influenza virus. Since then, reports have been issued that antisense molecules also inhibited oncogene expression and AIDS infection. Since antisense oligonucleotides specifically control the expression of undesirable genes, they have become one of the most promising fields as medicines in recent years.

The antisense method is based on the concept of inhibiting the process of translation from mRNA into protein in a series of information flow steps of the so-called central dogma, DNA → mRNA → protein, by use of an antisense oligonucleotide complementary to mRNA, thereby controlling the function of the DNA. The antisense method is a method which forms a double strand with single-stranded mRNA, the cause of disease, with the use of an antisense oligonucleotide introduced from outside, thereby inhibiting translation into its protein.

When a natural type oligonucleotide was applied as an antisense molecule to this method, however, the problem arose that it underwent degradation by various nucleases present in vivo, or its cell membrane permeation was not high. Thus, numerous nucleic acid derivatives or analogues have been synthesized, and have been extensively studied. For example, phosphorothioates having an oxygen atom on a phosphorus atom substituted by a sulfur atom, and methylphosphonates having an oxygen atom on a phosphorus atom substituted by a methyl group were synthesized. Recently, the derivatives or analogues having the phosphorus atom also substituted by a carbon atom, those having the structure of the sugar portion converted, or those having a nucleic acid base modified have also been synthesized. However, none of these derivatives or analogues are fully satisfactory in stability within cells, ease of synthesis, and binding specificity for sequences (the property of selectively controlling only the expression of particular genes).

Japanese Patent Application Laid-Open No. 1998-304889 discloses a nucleotide analogue unit modified in the sugar portion of an oligonucleotide constituting an antisense. This nucleotide analogue unit has a structure represented by the following formula 1 in which the sugar conformation is fixed in the N type

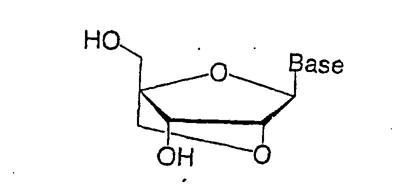

In this publication, an oligonucleotide derivative incorporating one or more nucleotide analogue units having the above-described sugar portion is synthesized, and the fundamental physical properties of the oligonucleotide derivative are measured extracellularly. That is, the oligonucleotide derivative and a sense strand comprising natural DNA or RNA are subjected to annealing, and the melting temperature of the annealing product is measured to investigate double strand forming capacity. Moreover, the resistance of this oligonucleotide derivative to nuclease enzymes is measured in vitro.

However, this publication does not disclose whether or not the oligonucleotide derivative shows nuclease resistance intracellularly as in the results of extracellular experiments to act stably as an antisense, or whether or not the oligonucleotide derivative binds to a naturally occurring gene particularly within cells to form a double strand or a triple strand, thereby becoming able to inhibit the expression of a particular gene actually.

The discovery of hepatitis C virus (HCV), on the other hand, is relatively recent, and a gene of HCV was isolated in 1988 (Choo, Q, L. et al., Science, 244, 359-362 (1989)). In connection with HCV, many reports have been made of an entire length base sequence and an amino acid sequence, a 5'-untranslated region, an internal ribosomal entry site (IRES), a stem region, etc. (Kato. N. et al., Proc. Natl. Acad. Sci. USA., 87, 9524-9528, (1990), Proc. Natl. Acad. Sci. USA., 88, 2451-2455, (1991), J. Virol., 65, 1105-1113, (1991), J. Gen. Virol., 72, 2697-2704, (1991), Virology, 188, 331-341, (1992), Tsukiyama. Kohara. et al., J. Virol., 66, 1476-1483, (1992), S. Tang. et al., J. Virol., 73, 2359-2364, (1999), Honda Masao et al., J. Virol., 73, 1165-1174, (1999), Honda Masao et al., RNA., 2(10), 955-968, (1996), Sasano T. et al., Genome Inf. Ser., 9, 395-396, (1998), Ito T. et al., J. Virol., 72, 8789-8796, (1998), Kamoshita N. et al., Virology., 233, 9-18, (1997), etc.). For HCV, interferon (IFN) has been said to be the only therapy. However, the severity of its side effects and its low rate of marked efficacy have been mentioned as major problems with treatment using IFN. Some reports say that IFN is effective in only 20% of patients. To increase its effectiveness, attempts such as a combination therapy with IFN and ribavirin have been applied. However, such attempts have not produced satisfactory results.

Aiming for gene therapy, techniques using the antisense method, ribozyme, and DNAzyme have been investigated, and in vivo studies on the antisense method and ribozyme are under way. Techniques concerned with antisenses, in particular, have widely propagated, and many reports on antisenses against HCV have been presented (WO 99/29350, WO 99/29350, WO 95/30746, WO 94/08002, Wakita et al., J. Biol. Chem., 269, 14205-14210, (1994), WO 94/24864). However, few antisenses satisfactory in enzyme resistance, affinity, and cytotoxicity have been obtained.

For the purpose of use in such an antisense method, it is desired to create an antisense oligonucleotide derivative, especially an antisense oligonucleotide derivative against HCV, which is minimally degraded by nucleases within cells, and binds to targeted HCV with high affinity, thus being capable of controlling and inhibiting, with high efficiency, the expression of its gene.

### [Disclosure of the Invention]

We, the inventors of the present invention, designed a nucleic acid analogue, in which the conformation of a sugar portion in the nucleic acid has been fixed, as a technique considered to be useful in the antisense method. We synthesized a nucleotide analogue unit serving as a unit structure for the nucleic acid analogue, and prepared an oligonucleotide derivative with the use of the nucleotide analogue unit. We have confirmed that this oligonucleotide derivative is very useful as an HCV antisense molecule.

### [BRIEF DESCRIPTION OF THE DRAWING]

FIG. 1 is a view showing the structure of an HCV genome.
FIG. 2 is an illustrative view showing the secondary structures of HCV-IRES.
FIG. 3 is an explanatory view showing the method of constructing a target plasmid containing HCV-IRES.
FIG. 4 is an explanatory view showing the method of constructing the target plasmid containing HCV-IRES.

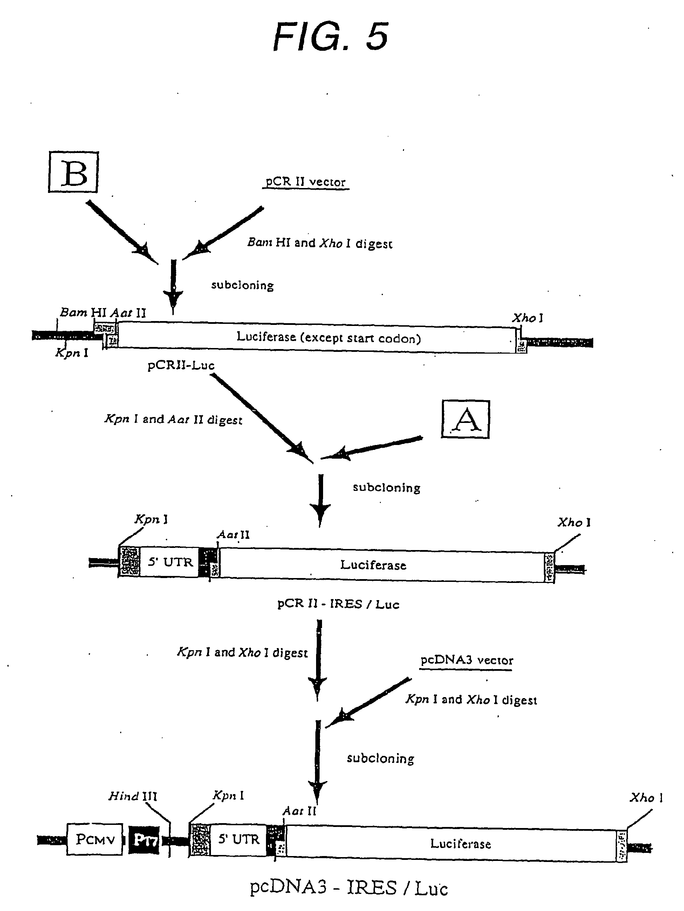

FIG. 5 is an explanatory view showing the method of constructing the target plasmid containing HCV-IRES.
FIG. 6 is a graph showing the HCV gene translation inhibiting effect of an antisense oligonucleotide derivative according to the present invention and a natural type antisense oligonucleotide which target the vicinity of the start codon of HCV-IRES in a non-cell system.
FIG. 7 is a graph showing the HCV gene translation inhibiting effect of an antisense oligonucleotide derivative according to the present invention and a natural type antisense oligonucleotide (different in length from those of FIG. 6) in a non-cell system.
FIG. 8 is a graph showing the HCV gene translation inhibiting effect of an antisense oligonucleotide derivative according to the present invention and a natural type antisense oligonucleotide (different in length from those of FIGS. 6 and 7) in a non-cell system.
FIG. 9 is a graph showing the HCV gene translation inhibiting effect of an antisense oligonucleotide derivative according to the present invention and a natural type antisense oligonucleotide which target the stem region of HCV-IRES in a non-cell system.
FIG. 10 is a view showing the structures of plasmids used in tests for investigating the HCV gene translation inhibiting effect of an antisense oligonucleotide in a cell system of Example 3.
FIG. 11 is a graph showing the HCV gene translation inhibiting effect of an antisense oligonucleotide derivative according to the present invention and a natural type antisense oligonucleotide, etc. which target the vicinity of the start codon of HCV-IRES in a cell system.
FIG. 12 is a graph showing the HCV gene translation inhibiting effect of an antisense oligonucleotide derivative according to the present invention (D-A159) and a natural type antisense oligonucleotide (BNA-A159) which target the stem region of HCV-IRES in a cell system.
FIG. 13 is a graph showing the HCV gene translation inhibiting effect of an antisense oligonucleotide derivative according to the present invention (BNA-A191) and a natural type antisense oligonucleotide (D-A191) which target the stem region of HCV-IRES in a cell system.

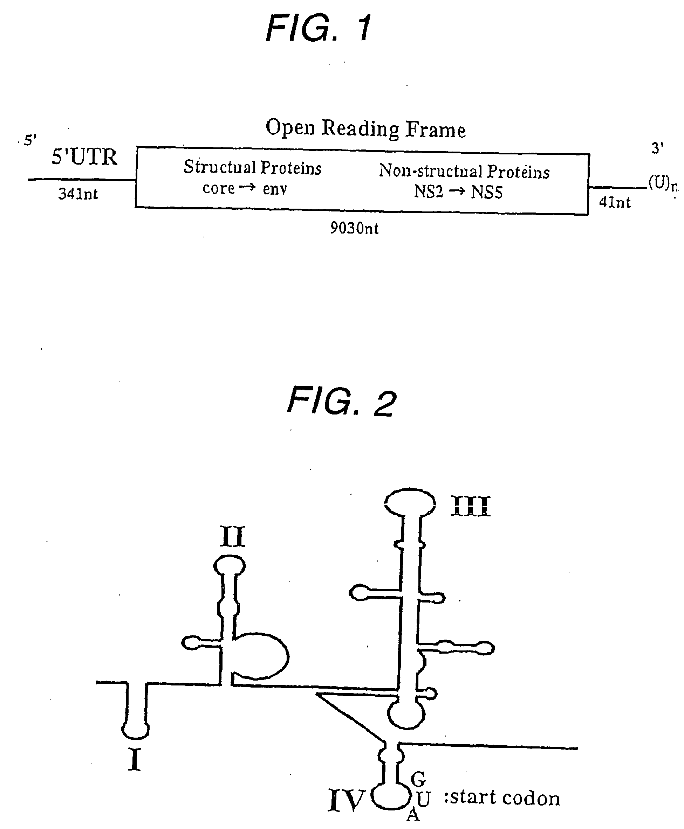

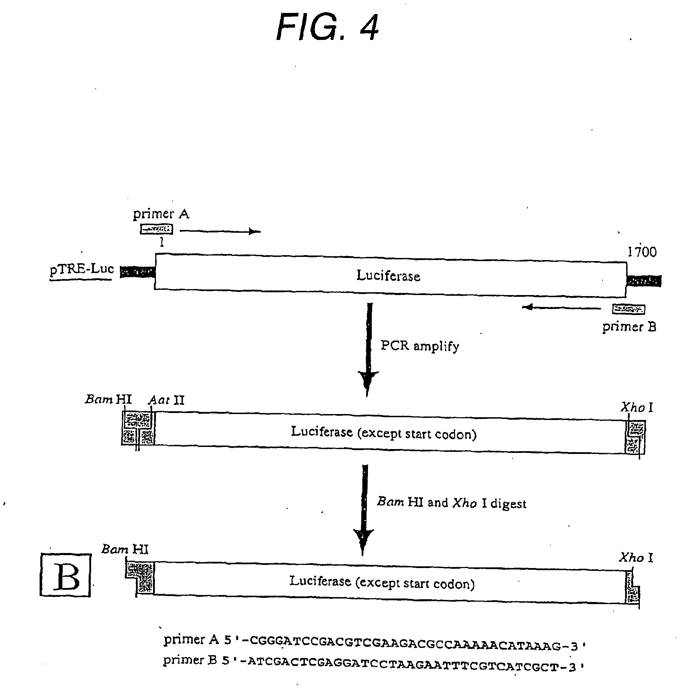

HCV is a single-stranded RNA virus, whose genome is about 9,500 bases in entire length, and which has one open reading frame in its inside (FIG. 4). This HCV-RNA is characterized in that translation starts in a cap-independent manner. For this purpose, it is required that ribosomes be bound to IRES (internal ribosomal entry site) within the 5' untranslated region (5'-UTR). This HCV-IRES is a highly conserved region having secondary structures as shown in FIG. 2, and has important functions. This is a region selected in the present invention as one of targets of the antisense method.

Thus, we synthesized antisense oligonucleotide derivatives, while setting, as an actual target, the vicinity of the start codon within IRES including the 5' untranslated region selected as a target of the antisense method against HCV. We confirmed these derivatives to have an excellent binding affinity and a sequence-specific marvelous antisense effect, and accomplished the present invention.

We also synthesized antisense oligonucleotide derivatives according to the present invention, targeted at a plurality of stem regions within HCV-IRES for which natural type oligonucleotides have shown no antisense effect. We confirmed the binding affinity and antisense effect of this derivative, and accomplished the present invention.

### [EMBODIMENTS OF THE INVENTION]

The oligonucleotide or polynucleotide derivative of the present invention has one or more nucleotide analogue units having a structure having a sugar conformation fixed, which is represented by the following general formula 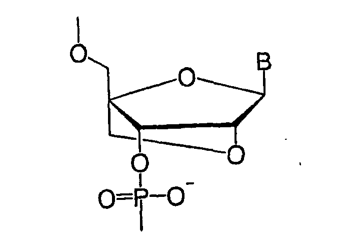 where B denotes a pyrimidine nucleic acid base or a purine nucleic acid base or an analogue thereof. Hereinafter, such an oligonucleotide derivative or a polynucleotide derivative will be referred to as a BNA oligonucleotide.

The oligonucleotide or polynucleotide derivative of the present invention is an oligonucleotide or polynucleotide derivative represented by the following general formula 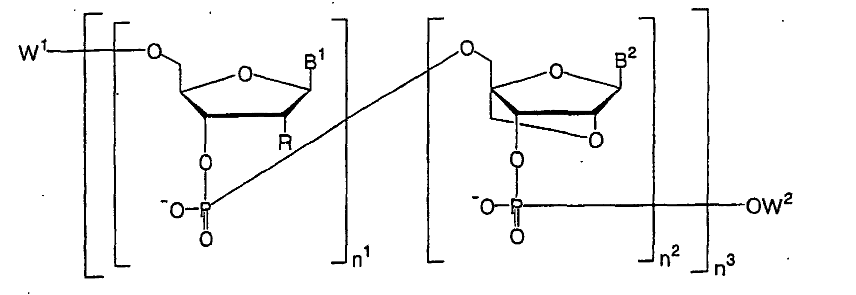 where B¹ and B² are the same or different, and each denote a pyrimidine nucleic acid base or a purine nucleic acid base or an analogue thereof; R represents hydrogen, a hydroxyl group, halogen, or an alkoxy group; W¹ and W² are the same or different, and each denote a natural type nucleotide, a synthetic nucleotide, or an oligonucleotide or polynucleotide, including any of these nucleotides, mediated by hydrogen, an alkyl group, an alkenyl group, an alkinyl group, a cycloalkyl group, an aralkyl group, an aryl group, an acyl group, a silyl group, a phosphate residue or a phosphodiester bond; n¹ or n² is the same or different, and denotes an integer of 0 to 50, provided that n¹ or n² is not zero at the same time and that all of n^{2'}s are not zero at the same time; and n³ denotes an integer of 1 to 50, provided that when n¹ and/or n² are or is 2 or more, B¹ and B need not be the same, and R's need not be the same.

There is no limitation on the number of the bases of the antisense oligonucleotide derivative according to the present invention, but this number is usually 5 to 50, preferably 9 to 30.

The pyrimidine nucleic acid base or purine nucleic acid base or analogue thereof in the present invention refers to thymine, uracil, cytosine, adenine, guanine or an analogue of any of these. As the analogues, purine nucleic acid base analogues and pyrimidine nucleic acid analogues can be named.

The purine nucleic acid base analogues can be selected preferably from the following compounds:
Guanosine diphosphate, 8-oxo-adenosine, 8-oxo-guanosine, 8-fluoro-adenosine, 8-fluoro-guanosine, 8-methoxy-adenosine, 8-methoxy-guanosine, 8-aza-adenosine, 8-aza-guanosine, azacytidine, fludarabine phosphate, 6-MP, 6-TG, azathioprine, allopurinol, acyclovir, ganciclovir, deoxyformicin, arabinosyladenine (ara-A), guanosine diphosphate-fucose, guanosine diphosphate-2-fluorofucose, guanosine diphosphate-βL-2-aminofucose, guanosine diphosphate-D-arabinose, and 2-aminoadenine.

The pyridine nucleic acid base analogues can be selected preferably from the following compounds:
5-fluorouracil, 5-chlorouracil, 5-bromouracil, dihydrouracil, 5-methylcytosine, 5-propynylthymine, 5-propynyluracil, 5-propynylcytosine, 5-fluorocytosine, fluoxyuridine, uridine, thymine, 3'-azido-deoxythymidine, 2-fluorodeoxycytidine, 3-fluoro-3'-deoxythymidine, 3'-dideoxycytidin-2'-ene, 3'-deoxy-3'-deoxythymidin-2'-ene, and cytosine arabinose.

The synthesis of the nucleotide analogue unit usable as an antisense in the present invention, and the synthesis of the oligonucleotide derivative containing the nucleotide analogue unit are described in detail in the aforementioned Japanese Patent Application Laid-Open No. 1998-304889. These descriptions are to be included herein.

The antisense oligonucleotide derivative against HCV in the present invention may be any oligo- or polynucleotide, if it can bind to HCV, inhibiting or suppressing the expression of its gene. It suffices that one or more of its nucleotides, its constituent units, are substituted by the nucleotide analogue units of the present invention. Examples of the sequence of the antisense binding to HCV are sequences complementary to the base sequence of HCV, and sequences which hybridize with the base sequence of HCV under stringent conditions.

Examples of the antisense oligonucleotide derivative are those against the vicinity of the start codon within IRES including the 5' untranslated region of HCV-RNA, and those against the stem region of HCV-RNA. Others which target various regions permitting the object of the present to be attained can be used. For example, those against exon or those against intron are named. More concretely, the following antisense base sequences against HCV are included:

### (Sequences targeting the vicinity of the translation start codon within IRES)

CTTTGAGGTTTAGGATTCGTGCTCATG (A367)

AGGTTTAGGATTCGTGCTCATG (A362)

TAGGATTCGTGCTCATG (A357)

### (Sequences targeting the stem region within IRES)

CCGGTTCCGCAGACCACTAT (A159)

ACCCGGTCGTCCTGGCAATT (A191)

As the antisense against HCV according to the present invention, there can be named sequences in which one or more bases of the antisense sequences against HCV represented by the above sequences have been substituted by the nucleotide analogue units of the present invention; antisense oligonucleotide derivatives containing these sequences; and oligonucleotide derivatives which hybridize under stringent conditions with DNA or RNA as a sequence complementary to the above antisense sequences. Hybridization is a well known technique (Sambrook, J. et al., Molecular Cloning 2nd ed., Cold Spring Harbor Lab. Press, 1989, etc.).

The stringent conditions, referred to in the present invention, can be selected, as desired, by people skilled in the art. An example is low stringent conditions. The low stringent conditions refer, for example, to 42°C, 0.1×SSC, 0.1% SDS, preferably 50°C, 0.1×SSC, 0.1% SDS, in washing after hybridization. More preferred stringent conditions are high stringent conditions. The high stringent conditions include 65°C, 6×SSC, 0.1% SDS.

The oligonucleotide derivatives of the present invention have also been confirmed to show excellent nuclease resistance as compared with natural type oligonucleotides having the same base sequence. Thus, the oligonucleotide derivatives of the present invention are advantageous in that even when administered into cells, they are stable, and their excellent antisense effect persists (T. Imanishi, S. Obika, J. Synth. Org. Chem., 11, 969 (1999)).

The antisense oligonucleotide derivatives of the present invention essentially require the use of the nucleotide analogue unit having only the sugar portion modified. It is permissible to use the nucleotide analogue unit in which in addition to modification of the sugar portion, other portion, for example, the phosphodiester portion has been modified, as in phosphorothioates. It is also possible to construct oligonucleotide derivative antisenses by combining the nucleotide analogue unit of the present invention having a modified sugar portion with other nucleotide analogue units as publicly known antisense constituent units.

The antisense oligonucleotide derivatives of the present invention can be mixed with suitable base materials inert thereto, whereby preparations for external use, such as liniments and cataplasms, can be formed. If desired, vehicles, tonicity agents, solution adjuvants, stabilizers, preservatives, and soothing agents are added to the antisense oligonucleotide derivatives, whereby tablets, powders, granules, capsules, liposome capsules, injections, liquids and solutions, nasal drops, and lyophilized products can be formed. These preparations can be produced by the usual methods.

The antisense oligonucleotide derivatives of the present invention are directly administered to the lesion of the patient, or administered into the blood vessel so that they can eventually reach the lesion. Furthermore, antisense encapsulation materials for enhancing prolonged action or membrane permeation can also be used. For example, liposome, poly-L-lysine, lipid, cholesterol, lipofectin or an analogue of any of these can be named.

The dose of the antisense oligonucleotide derivative of the present invention may be a preferred amount adjusted, as desired, depending on the condition, age, sex and body weight of the patient. The mode of administration may depend on the condition of the patient, the dosage form, etc., and may be a preferred method selected, as desired, from various methods of administration, such as oral administration, intramuscular administration, intraperitoneal administration, intradermal administration, subcutaneous administration, intravenous administration, intraarterial administration, and rectal administration.

The antisense oligonucleotide derivatives against HCV according to the present invention show various excellent characteristics as antisense DNA's. This will be described in detail by the following Examples. Example 1: Double strand forming capacity of oligonucleotide derivatives containing nucleotide analogue unit of the present invention by measurement of melting temperature
(1) With the vicinity of the start codon (342nt-344nt) within HCV-IRES being contemplated as a target, antisense oligonucleotides having the sequences indicated below were designed and constructed. To investigate the relation between the length of the antisense oligonucleotide and the antisense effect, 5 bases and 10 bases/were cut off from oligonucleotide A-367, beginning at the 5'-terminal, to synthesize A362 and A357, respectively. CTTTGAGGTTTAGGATTCGTGCTCATG (A367) AGGTTTAGGATTCGTGCTCATG (A362) TAGGATTCGTGCTCATG (A357)
   In connection with these oligonucleotides of various lengths, moreover, BNA oligonucleotides (BNA-oligo) and phosphorothioate type oligonucleotides (S-oligo) were prepared in addition to natural type oligonucleotides (D-oligo). In regard to A367, for example, D-A367 (natural type), BNA-A367 (BNA type) and S-A367 (phosphorothioate type) were prepared.
   The sequences of the synthesized oligonucleotides were as follows: 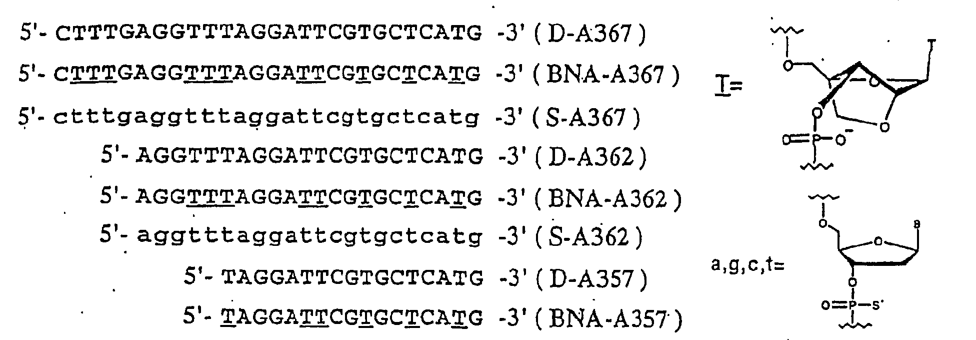
5'- CTTTGAGGTTTAGGATTCGTGCTCATG -3' ( D-A367 )
5'- CTTTGAGGTTTAGGATTCGTGCTCATG -3' ( BNA-A367 )    T=
5'- ctttgaggtttaggattcgtgctcatg -3' ( S-A367 )
5'- AGGTTTAGGATTCGTGCTCATG -3' ( D-A362 )
5'- AGGTTTAGGATTCGTGCTCATG -3' ( BNA-A362 )
5'- aggtttaggattcgtgctcatg -3' ( S-A362 )
5'- TAGGATTCGTGCTCATG -3' ( D-A357 )    a,g,c,t=
5'- TAGGATTCGTGCTCATG -3' ( BNA-A357 )
(2) To investigate the binding affinity of these synthesized antisense oligonucleotides for complementary strand RNA, their Tm values (melting temperatures) were measured.
   The above synthesized natural type oligonucleotides and oligonucleotide derivatives were used as antisense strands, and annealed to sense strands comprising natural type DNA or RNA. The melting temperatures (Tm values) of the annealing products were measured to examine the hybridizing capacity of the oligonucleotide derivatives of the present invention for complementary DNA and RNA.
   A sample solution (500 µl) with end concentrations of NaCl 100 mM, sodium phosphate buffer (pH 7.2) 10 mM, antisense strand 1 µM, and sense strand 1 µM was bathed in boiling water, and cooled slowly to room temperature over 10 hours. While a nitrogen stream was passed into a cell chamber of a spectrophotometer (Beckman DU650) for prevention of dew formation, the sample solution was gradually cooled to 5°C, and further held at 5°C for 20 minutes, whereafter measurement was started. The temperature of the sample was raised to 95°C at a rate of 0.5°C/minute, and ultraviolet absorption at 260 nm was measured at intervals of 0.5°C. The intensity of the ultraviolet absorption greatly increased at particular temperatures. The temperature at the mid-point of this transition was the melting temperature, i.e. Tm value. The higher the binding affinity, the higher the Tm value.
   The results are shown in the following table. In the nucleotide sequences, the nucleotide analogue units of the present invention are underlined. 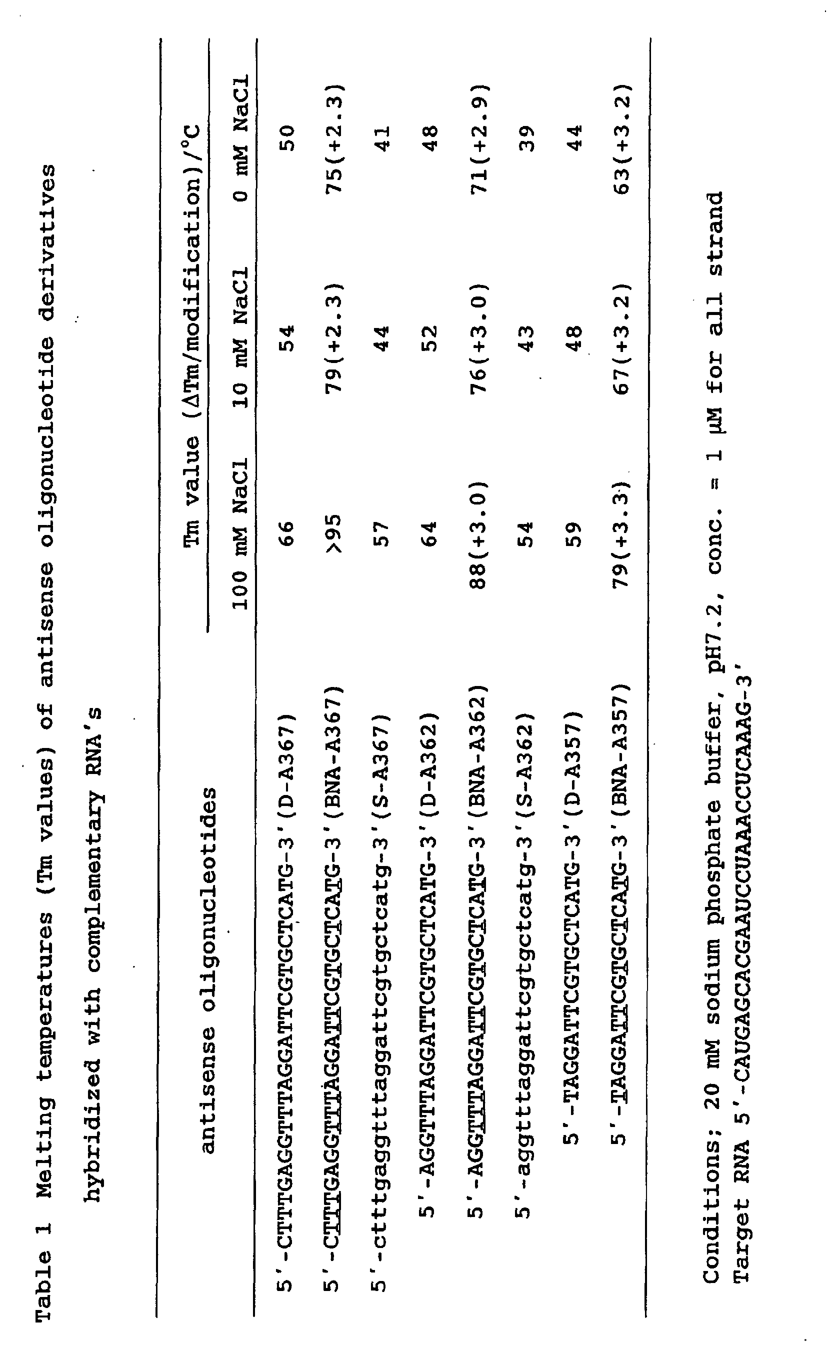
(3) Natural type antisense oligonucleotide (D-oligo) and the oligonucleotide derivative (BNA-oligo) of the present invention, targeting the stem region within HCV-IRES, were synthesized in the same manner as in (1) stated above. In the nucleotide sequences of the following formulas, the nucleotide analogue units of the present invention are underlined.

CCGGTTCCGCAGACCACTAT (D-A159)

CCGGTTCCGCAGACCACTAT (BNA-A159)

The Tm values were measured under the same conditions as for the above-mentioned antisense oligonucleotides targeting HCV-IRES.

The results are shown in Table 2 below.

**Table 2**

| Test | Antisense molecule | Tm value (ΔTm/mod.)/°C | |
|---|---|---|---|
| | | 100 mM NaCl | 0 mM NaCl |
| 1. | D-A159 | 69 | 54 |
| 2. | BNA-A159 | >95 | 84 (+3.0) |

As clear from Table 1 and Table 2, the s-oligo's showed decreases in the Tm values, i.e. decline of binding affinity, in comparison with the D-oligo's, while the BNA-oligo's showed marked increases in the Tm values. These findings confirmed that the BNA-oligo's had very high binding affinity for complementary strand RNA as compared with the S-oligo's and the D-oligo's. This outcome was confirmed in the case of the start codon target and the stem region target. In connection with the start codon target, the same results were noted for the oligonucleotides with decreased lengths.

### Example 2: HCV gene translation inhibiting effect of oligonucleotide derivatives in non-cell system

In this Example, the antisense effect against HCV gene in a non-cell system was examined. Target plasmid pcDNA3-IES/Luc containing IRES (internal ribosomal entry site) within the 5' untranslated region of hepatitis C virus was designed and constructed. This target plasmid was used to check for the antisense effect of oligonucleotide derivatives.

### (Construction of plasmid pcDNA3-IES/Luc)

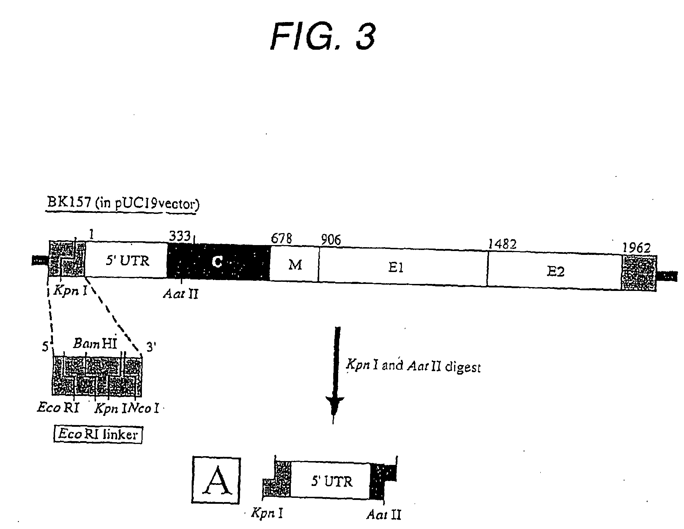

In accordance with the method of Caselmann (Caselmann et al., Hepatology, 22, 707 (1995)), BK157 (A. Takamizawa et al., J. Virol., 65, 1105 (1991)), a clone DNA of HCV, was digested with KpnI and AatII to prepare a fragment A containing the target site (see FIG. 3).

Then, a fragment B containing a luciferase gene (Luc) to be used as a reporter gene was prepared (see FIG. 4). This step was performed by amplifying Luc by PCR of pTRE-Luc with the use of a primer A and a primer B, and then digesting the amplification product with BamHI and XhoI.

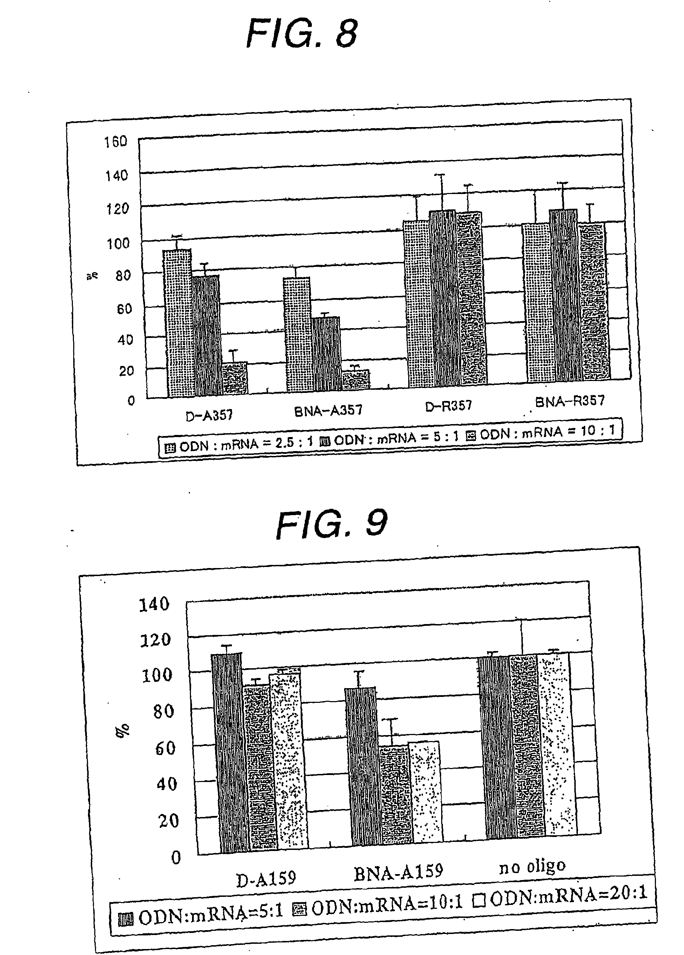

As a problem with incorporation of these fragments A and B into a pcDNA3 vector, it was expected that the incorporation of the fragment B would not be performed, since many recognition sequences for AatII are present in pcDNA. Thus, it was planned that both the fragments A and B would be incorporated into a pCRII vector, and then the vector would be replaced by pcDNA3 at the final stage to obtain the desired plasmid (see FIG. 9).

First, a pCRII vector (a product of Invitrogen) was digested with BamHI and XhoI, and then the digest was ligated to the fragment B containing the luciferase gene, thereby obtaining pCRII-Luc. The resulting pCRII-Luc was digested with KpnI and XhoI, and the digest was ligated to the fragment A containing the target site, thereby obtaining pCRII-IRES/Luc.

Finally, in order to replace the vector of pCRII-IRES/Luc by pcDNA3 having promoters (promoters CMV and T7) capable of acting in both of a non-cell system and a cell system, the pCRII-IRES/Luc and a pcDNA3 vector (a product of Invitrogen) were digested with KpnI and XhoI. Then, the digests were ligated together, successfully preparing the desired target plasmid pcDNA3-IRES/Luc. Using this plasmid, the antisense effect against HCV was evaluated by the following experiments:

### (HCV gene translation inhibiting effect in non-cell system)

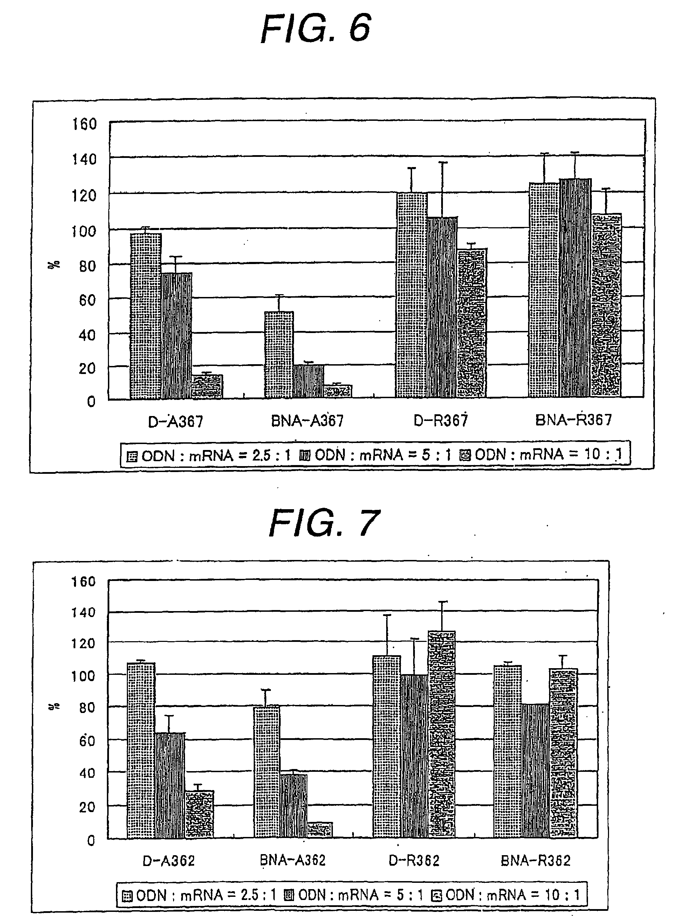

(1) In expectation of an increase in the efficiency of in vivo transcription reaction of the target plasmid pcDNA3-IES/Luc containing IRES (internal ribosomal entry site) within the 5' untranslated region of hepatitis C virus, the restriction enzyme XhoI was used for linearization. The resulting linearized DNA was used for in vitro transcription reaction to synthesize mRNA. This mRNA was converted into a protein by an in vitro translation reaction using an oligonucleotide and a rabbit reticulocyte solution (RRL), and then luciferase assay was performed.
   Initially, a study was conducted using an antisense oligonucleotide targeting the vicinity of the start codon. In this study, D-oligo and BNA-oligo were used. The antisense oligo's (D-A367, BNA-A367) for D-oligo and BNA-oligo, and random oligo's (D-R367, BNA-R367) as negative controls were each added such that the ratio of their concentration to the concentration of mRNA would be 2.5:1, 5:1 and 10:1, whereafter a translation reaction was carried out. The resulting protein was used for luciferase assay. The results are shown in FIG. 6, with the outcome for the translation reaction performed without addition of the oligonucleotide being taken as 100%.
   The results confirmed that the expression of luciferase was suppressed by the addition of the antisense oligonucleotide, and this suppression of expression was augmented as the concentration of the oligonucleotide added was increased, thus showing a concentration-dependent antisense effect.
   The antisense effect was observed in each of D-A367 and BNA-A367. However, BNA-A367 showed a better effect of suppressing gene expression at any of the concentrations, and proved to be a better antisense oligonucleotide. No gene expression suppressing effect was observed in the random oligonucleotide (BNA-R367) used as the control. Thus, this antisense effect was confirmed to be sequence-specific.
(2) Then, in order to examine changes in the antisense effect by decreasing the length of the oligonucleotide, the same tests as described above were conducted using the D-oligo and BNA-oligo of each of A362 which was decreased by 5 bases from A-367 at its 5' side, and A357 which was decreased by 10 bases from A-367 at its 5' side.
   The results with the respective oligonucleotides are shown in FIGS. 7 and 8, with the outcome for the translation reaction performed without addition of the oligonucleotide being taken as 100%.
   The results shown in FIGS. 7 and 8 confirmed that the BNA antisense oligonucleotides showed an excellent gene expression suppressing effect, even though they had decreased lengths. Particularly, even at the low concentrations at which the D-oligo's did not show a high antisense effect, the BNA-oligo's were confirmed to produce a significant antisense effect. Neither BNA-R362 nor BNA-R357 showed a gene expression suppressing effect. Thus, the antisense effect was confirmed to be sequence-specific.
(3) The same tests were conducted on D-A159 and BNA-A159 targeting the stem region within HCV-RNA. Each oligonucleotide was added such that the ratio of its concentration to the concentration of mRNA would be 5:1, 10:1 and 20:1, whereafter a translation reaction was performed. The results of luciferase assay are shown in FIG. 9, with the outcome for the translation reaction performed without addition of the oligonucleotide being taken as 100%.

When D-A159 was used, no gene expression suppressing effect was observed at any of the concentrations. The reason may be that the binding force of the D-oligo was too weak to strip off the intramolecular hydrogen bond of the stem region for binding to the stem region.

When BNA-A195 was used, on the other hand, it showed a gene expression suppressing effect of about 50% when its ratio to mRNA was 10:1 and 20:1. This may be attributed to the potent binding affinity of the BNA oligonucleotide of the present invention taking effect.

### Example 3 HCV gene translation inhibiting effect of BNA antisense oligonucleotide in cell system

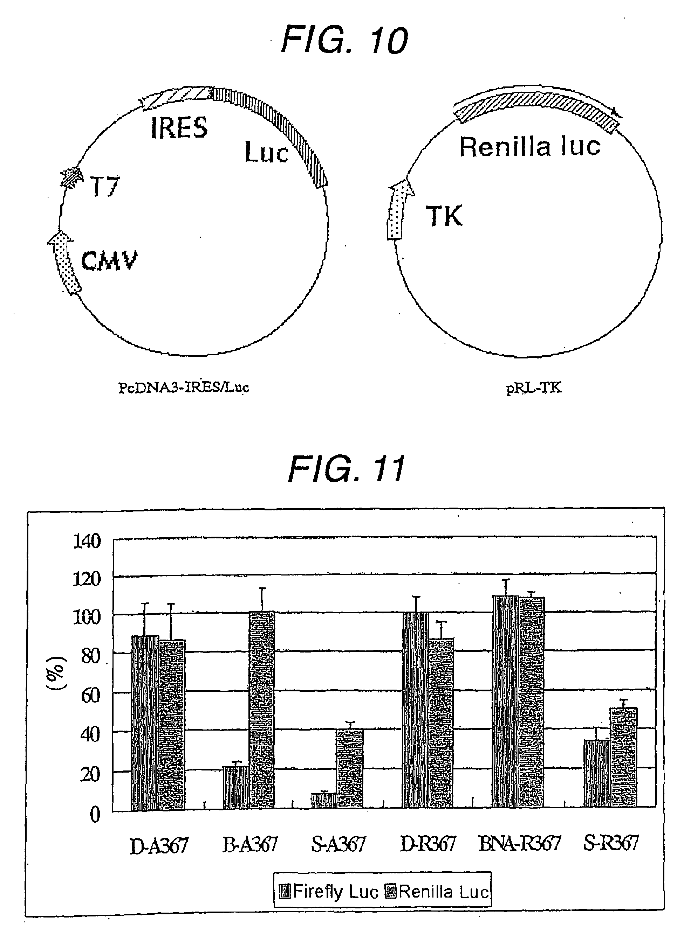

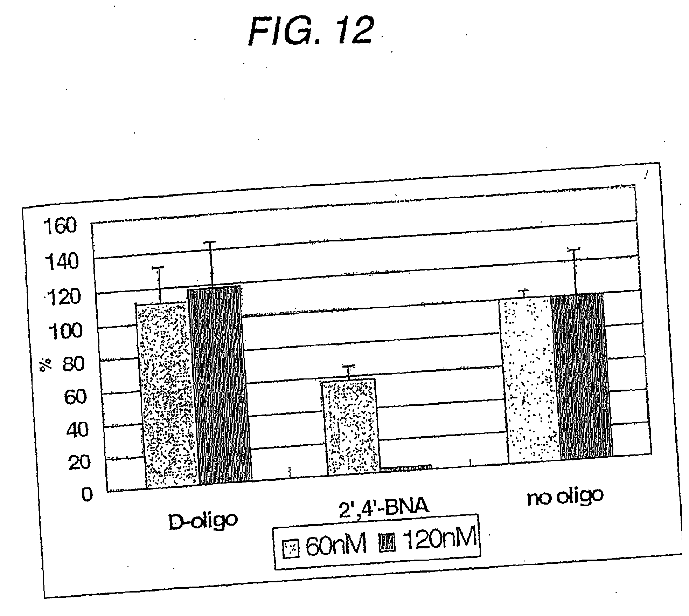

The HCV gene translation inhibiting effect was investigated in a cell system with the use of HepG2 cells (provided by Osaka University Faculty of Pharmacy), a cell strain of the hepatic cancer origin.
(1) HepG2 cells were seeded onto each well in the same amount of 5×10⁴ cells, and incubated for 24 hours. Then, an oligonucleotide, the target plasmid pcDNA3-IRES/Luc, and pRL-TK were transfected using a lipofect amine in the absence of serum. The pRL-TK, transfected together with the target plasmid, is a plasmid free from HCV-IRES, the target site. This plasmid was used to investigate the toxicity of the oligonucleotide added, namely, its nonspecific expression suppressing effect. The pRL-TK contains the luciferase gene of a sea pansy (renilla) as a reporter gene. By performing dual luciferase assay, the pRL-TK enables assay to be performed separately from the luciferase gene of a firefly, which is the reporter gene of the target plasmid pcDNA3-IRES/Luc (FIG. 10). That is, the one which suppresses the expression of renilla luciferase free of the target shows a nonspecific gene expression suppressing effect.
   Transfection in Opti-MEM was performed for 4 hours in the absence of serum, and then the culture medium was replaced by DMEM where serum was present. Then, incubation was performed for 20 hours, and the cells were harvested and subjected to dual luciferase assay.
(2) First, a study was made of antisense oligonucleotides targeting the start codon. The oligonucleotides used in tests were 27mer antisenses and random oligonucleotides for D-oligo, BNA-oligo and S-oligo, which were added at end concentrations of 120 nM.
   The results are shown in FIG. 11, with the outcome for transfection of the plasmid performed without addition of the oligonucleotide being taken as 100%.
   As shown in the results of FIG. 11, D-A367, the D-oligo that produced an antisense effect in the non-cell system, showed no gene expression suppressing effect in this test in the cell system. This may be because the D-oligo was degraded by nucleases present in the cells. In connection with the S-oligo evaluated to have excellent enzyme resistance, the expression of firefly luciferase was markedly suppressed by S-367. However, the S-oligo poses the problem of toxicity, and was thus shown to have a nonspecific expression suppressing effect. That is, the suppression of firefly luciferase expression was observed in the random oligonucleotide S-R367 which theoretically does not suppress expression. Additionally, S-A367 and S-R367 also suppress the expression of renilla luciferase free of the target sequence.
   In the case of the BNA oligonucleotide, BNA-A367 showed an excellent effect of suppressing the gene expression of firefly luciferase, and did not affect the expression of renilla luciferase. The random oligonucleotide BNA-R367 showed no gene expression suppressing effect. In the light of these results, it is believed that the effect of the BNA antisense oligonucleotide is a sequence-specific effect, which is ascribed to the excellent binding affinity and enzyme resistance of the BNA oligonucleotide. Thus, it has become clear that only the BNA oligonucleotide shows an excellent antisense effect even within cells.
(3) Next, a study was made of the intracellular antisense effect of BNA-A159 targeting the stem region within HCV-IRES. The oligonucleotides used were D-A159 and BNA-A159, and they were transfected at end concentrations of 60 nM and 120 nM. The results are shown in FIG. 12, with the outcome for transfection performed without addition of the oligonucleotide being taken as 100%.
   As shown in the results of FIG. 12, D-A159 showed no expression suppressing effect as in the non-cell system, and was confirmed to produce no antisense effect. BNA-A159, on the other hand, produced suppression of about 50% at 60 nM, and nearly completely suppressed the expression of luciferase at a concentration of 120 nM. Thus, BNA-A159 was confirmed to show an excellent antisense effect.
(4) A natural type antisense oligonucleotide (D-oligo) and the oligonucleotide derivative of the present invention (BNA-oligo) having the following sequences, which target the stem region within HCV-IRES, were synthesized in the same manner as for D-A159 and BNA-A159. In the nucleotide sequences of the following formulas, the nucleotide analogue units of the present invention are underlined.

ACCCGGTCGTCCTGGCAATT (D-A191)

ACCCGGTCGTCCTGGCAATT (BNA-A191)

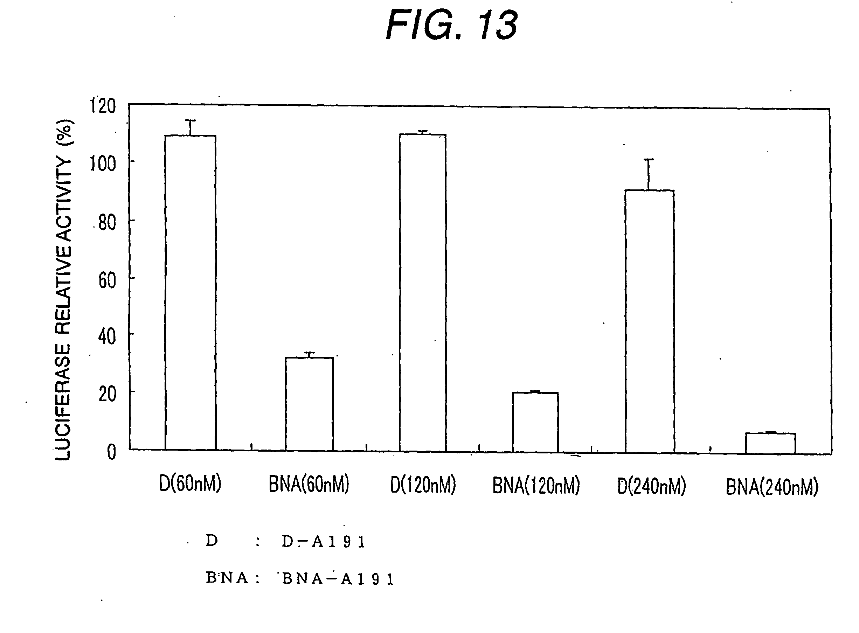

The intracellular antisense effect of BNA-A191 was studied similarly to BNA-A159. D-A191 and BNA-A191 were used and transfected at end concentrations of 60 nM, 120 nM and 240 nM. The results are shown in FIG. 13, with the outcome for transfection performed without addition of the oligonucleotide being taken as 100%.

As shown in the results of FIG. 13, D-191 showed no expression suppressing effect, and was confirmed to produce no antisense effect.

BNA-A191, on the other hand, was confirmed to show an excellent antisense effect.

The above remarkable effect of the BNA antisense oligonucleotide in the cell system as compared with the non-cell system is presumed to be ascribed to its excellent enzyme resistance and the high intracellular concentration of ribonucleases.

### [INDUSTRIAL APPLICABILITY]

The BNA antisense oligonucleotides of the present invention have excellent binding affinity for HCV-RNA, show high enzyme resistance, and low in cytotoxicity. Thus, they show a specific HCV gene expression suppressing effect in both of a non-cell system and a cell system. The BNA antisense oligonucleotides of the present invention are also effective in an antisense method targeting HCV, no matter what secondary structures, such as loops or stems, in a target RNA nucleic acid are. Hence, the BNA antisense oligonucleotides of the present invention are promising as therapeutics for gene therapy of HCV for which there have been no potent therapeutic drugs except interferon.

[SEQUENCE LISTING]
SEQUENCE LISTING
<110> Takeshi Imanishi
<120> Novel Antisense Oligonucleotide Analogues against HCV Gene
<130> 011935
<160> 10

<210> 1
<211> 27
<212> DNA
<212> Artificial Sequence
<220> antisense against HCV gene
<221>
<222>
<223>
<400> 1
ctttgaggtttaggattcgtgctcatg

<210> 2
<211> 22
<212> DNA
<212> Artificial Sequence
<220> antisense against HCV gene
<221>
<222>
<223>
<400> 2

aggtttaggattcgtgctcatg

<210> 3
<211> 17
<212> DNA
<212> Artificial Sequence
<220> antisense against HCV gene
<221>
<222>
<223>
<400> 3
taggattcgtgctcatg

<210> 4
<211> 20
<212> DNA
<212> Artificial Sequence
<220> antisense against HCV gene
<221>
<222>
<223>
<400> 4
ccggttccgcagaccactat

<210> 5
<211> 35
<212> DNA

<212>    Artificial Sequence
<220>    PCR primer
<221>
<222>
<223>
<400>  5
cgggatccgacgtcgaagacgccaaaaacataaag

<210>    6
<211>    35
<212>    DNA
<212>    Artificial Sequence
<220>    PCR primer
<221>
<222>
<223>
<400>  6
atcgactcgaggatcctaagaatttcgtcatcgct

<210>    7
<211>    27
<212>    DNA
<212>    Artificial Sequence
<220>    modified base
<221>
<222>
<223>

<400>  7
cnnngaggnnnagganncgngcncang

<210>  8
<211>  22
<212>  DNA
<212>  Artificial Sequence
<220>  modified base
<221>
<222>
<223>
<400>  8
aggnnnagganncgngcncang

<210>  9
<211>  17
<212>  DNA
<212>  Artificial Sequence
<220>  modified base
<221>
<222>
<223>
<400>  9
nagganncgngcncang

<210>  10

<211>    15
<212>    DNA
<212>    Artificial Sequence
<220>    modified base
<221>
<222>
<223>
<400>   10
nnggnnnngnagaccannan

<210>    11
<211>    20
<212>    DNA
<212>    Artificial Sequence
<220>    antisense against HCV gene
<221>
<222>
<223>
<400>   11
acccggtcgtcctggcaatt

<210>    12
<211>    20
<212>    DNA
<212>    Artificial Sequence
<220>    modified base
<221>

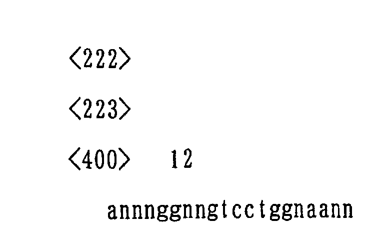

## Claims

1. An antisense oligonucleotide derivative against hepatitis C virus, which contains one or more nucleotide analogue units having a modified sugar portion.

2. The antisense oligonucleotide derivative according to claim 1, wherein said nucleotide analogue unit having a modified sugar portion has a structure represented by the following general formula 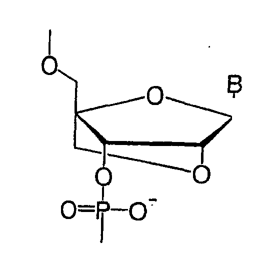 where B denotes a pyrimidine or purine nucleic acid base or an analogue thereof.

3. The antisense oligonucleotide derivative according to claim 1 or 2, which targets a 5' untranslated region of hepatitis C virus RNA.

4. The antisense oligonucleotide derivative according to claim 3, wherein said 5' untranslated region is an internal ribosomal entry site (IRES).

5. The antisense oligonucleotide derivative according to claim 1 or 2, which targets a stem region of hepatitis C virus RNA.

6. The antisense oligonucleotide derivative according to any one of claims 1 to 5, which contains any one of the following base sequences:

CTTTGAGGTTTAGGATTCGTGCTCATG

AGGTTTAGGATTCGTGCTCATG

TAGGATTCGTGCTCATG

CCGGTTCCGCAGACCACTAT

ACCCGGTCGTCCTGGCAATT

7. The antisense oligonucleotide derivative according to claims 1 to 5, containing an oligonucleotide derivative which hybridizes with DNA or RNA of a sequence complementary to any one of the base sequences of claim 6 under stringent conditions.

8. An anti-hepatitis C virus agent containing the antisense oligonucleotide derivative against hepatitis C virus RNA according to any one of claims 1 to 7 as an active ingredient.
